(19) Europäisches Patentamt
European Patent Office
Office européen des brevets

(11) Publication number: **0 025 498**
**A2**

# EUROPEAN PATENT APPLICATION

(21) Application number: **80104446.2**

(22) Date of filing: **28.07.80**

(51) Int. Cl.³: **C 07 D 237/24**

(30) Priority: **30.07.79 US 61588**

(43) Date of publication of application: **25.03.81**
**Bulletin 81/12**

(84) Designated Contracting States: **AT BE CH DE FR GB IT LI NL SE**

(71) Applicant: **Rohm and Haas Company, Independence Mall West, Philadelphia, Pennsylvania 19105 (US)**

(72) Inventor: **Carlson, Glenn Richard, 305 Britt Road, N. Wales, Pa. (US)**

(74) Representative: **Deufel, Paul et al, Patentanwälte MÜLLER-BORé-DEUFEL SCHÖN-HERTEL Siebertstrasse 4, D-8000 München 86 (DE)**

(54) **Process for the preparation of pyridazine derivatives.**

(57) A 1-aryl-1,4-dihydro-4-oxopyridazine carboxylic acid ester of the formula:

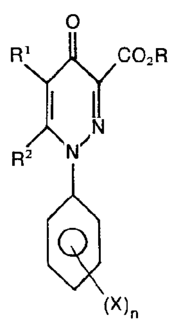

(I)

is prepared by an improved process which comprises reacting, in a liquid medium and in the substantial absence of water available to undergo reaction to form undesired by-product(s), an alcohol with a hydrazone in the presence of (a) a strong acid catalyst and (b) a desiccant which serves to remove water of reaction. The alcohol used is of the formula ROH and the hydrazone used is of the formula:

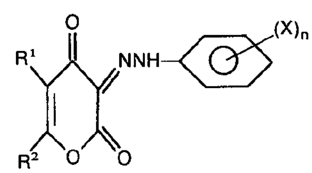

In the above formulae:

R is $(C_1–C_6)$alkyl, $(C_1–C_4)$alkoxy$(C_1–C_4)$alkyl, $(C_3–C_8)$-cycloalkyl, $(C_3–C_8)$cycloalkyl$(C_1–C_4)$alkyl, halo$(C_1–C_6)$-alkyl, benzyl or benzyl the ring of which is substituted with up to two of the same or different substituents selected from chloro, bromo, methyl, ethyl, methoxy, cyano, nitro and trifluoromethyl;

$R^1$ and $R^2$ are selected from hydrogen and $(C_1–C_4)$-alkyl;

X is selected from halo, trihalomethyl, nitro, $(C_1–C_4)$-alkyl, $(C_1–C_4)$alkoxy, $(C_1–C_4)$alkylthio, $(C_1–C_4)$alkylsulfinyl, $(C_1–C_4)$alkylsulfonyl, cyano and carboxy, the X substituents being the same or different when n is >1, and n is 0 or an integer of from 1–3.

The esters of Formula I find use as plant growth regulators.

EP 0 025 498 A2

PATENTANWÄLTE
MÜLLER-BORÉ · DEUFEL
SCHÖN · HERTEL
8 MÜNCHEN 86 · SIEBERTSTR. 4
TEL. (089) 47 40 05 · TELEX 5-24 85



0025498
R 1197 EU

# PROCESS FOR THE PREPARATION OF PYRIDAZINE DERIVATIVES

This invention concerns an improved process for the preparation of pyridazine derivatives, namely esters of 1-aryl-1,4-dihydro-4-oxopyridazine-3-carboxylic acids.

The preparation of 1-aryl-1,4 dihydro-4-oxo-pyridazine-3-carboxylic acids via the rearrangement of a hydrazone in hot aqueous mineral acids or hot aqueous base is disclosed in Belgian Patent 864,704. These methods are additionally disclosed in British Patent Specification 762,141; in United States Patent 2,835,671, in Helvetica Chimica Acta, 39, 1741 (1956), in the Journal of the American Chemical Society (JACS), 78, 624 (1955) and in JACS, 70, 2253 (1948).

That 1-aryl-1,4-dihydro-4-oxypyridazine carboxylic acids can be esterified via Fischer esterification is disclosed in Helvetica Chimica Acta, 39, 1741 (1956) and United States Patent 2,835,671. The Fischer esterification method requires the isolation of the carboxylic acid prior to the esterification reaction. Fischer esterification reactions generally produce a crude product that is highly colored and rather impure. Several recrystallizations are often required to obtain material of adequate purity for analysis and testing. Fischer esterification generally requires several hours to several days before completion and gives low yields.

In accordance with the present invention, there is provided a process for the preparation of a 1-aryl-1, 4-dihydro-4-oxopyridazine carboxylic acid ester of the formula:

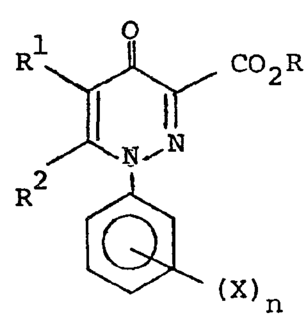

(I)

which comprises reacting, in a liquid medium and in the substantial absence of water available to undergo reaction to form undesired by-product(s), an alcohol with a hydrazone in the presence of (a) a strong acid catalyst and (b) a desiccant which serves to remove water of reaction, wherein the alcohol is of the formula ROH (II) and the hydrazone is of the formula:

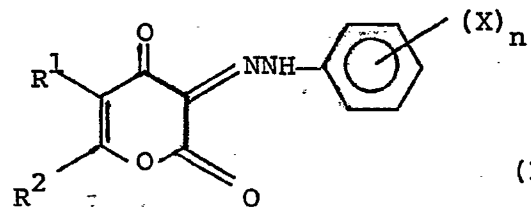

(III)

where in the above formulae:

R is $(C_1-C_6)$alkyl, $(C_1-C_4)$alkoxy$(C_1-C_4)$alkyl, $(C_3-C_8)$cycloalkyl, $(C_3-C_8)$cycloalkyl-$(C_1-C_4)$alkyl, halo$(C_1-C_6)$alkyl, benzyl or benzyl the ring of which is substituted with up to two of the same or different substituents selected from chloro, bromo, methyl, ethyl, methoxy, cyano, nitro and trifluoromethyl;

$R^1$ and $R^2$ are selected from hydrogen and $(C_1-C_4)$alkyl;

X is selected from halo, trihalomethyl, nitro, $(C_1-C_4)$alkyl, $(C_1-C_4)$alkoxy, $(C_1-C_4)$alkylthio, $(C_1-C_4)$alkylsulfinyl, $(C_1-C_4)$alkylsulfonyl, cyano and carboxy, the X substituents being the same or different when n is $>1$; and n is 0 or an integer of from 1-3.

The esters of Formula I are useful as plant growth regulants and in particular as chemical hybridization agents.

The reaction between the alcohol and the hydrazone is generally conducted at a temperature from 25°C to 150°C, preferably 40°C to 120°C and even more preferably 60°C to 110°C and is also generally conducted in the presence of an inert solvent. Usually at least one mole of the alcohol ROH will be used per mole of the hydrazone of Formula III.

The desiccant, which is used in at least an amount sufficient to remove water of reaction, may be a tri$(C_1-C_4)$alkylorthoformate, a tri$(C_1-C_4)$alkyl borate, a tetra$(C_1-C_4)$alkyl silicate, a $(C_1-C_4)$alkyl acetal, a $(C_1-C_4)$alkyl ketal, a molecular sieve or phosphorus pentoxide. The most preferred desiccant is a $(C_1-C_4)$alkylorthoformate.

The strong acid, which is used in a catalytic amount, may be sulfuric, hydrochloric, hydrobromic, nitric, perchloric, phosphoric, trifluoromethylacetic and methane sulfonic acid.

If transesterification is to be avoided, the alcohol chosen as the reactant ROH will be the same as that liberated by the tri$(C_1-C_4)$alkylorthoformate in acting as desiccant and the same as any alcohol used as a solvent.

The term "alkyl" as used in this specification is meant to include both branched and straight chain alkyl groups; also by the use of an unqualified term such as butyl, it is meant to include all the various isomers. The term "halo" means fluoro, chloro, bromo or iodo.

Typical alcohols of Formula (II) that can be utilized in the process of the invention include methanol, ethanol, isopropanol, n-propanol, n-butanol, sec-butanol, tert-butanol, n-pentanol, n-hexanol, methoxymethanol, cyclohexanol, cyclohexylmethanol, chloromethanol, benzyl alcohol and chlorobenzyl alcohol.

Typical desiccants that can be used include trimethylorthoformate, triethylorthoformate,

triisopropylorthoformate, tri-n-butylorthoformate, trimethylborate, triethylborate, tetramethylsilicate, tetraethylsilicate, acetaldehyde dimethyl acetal, acetone dimethyl ketal, Linde Type 4-A molecular sieves and phosphorus pentoxide.

Typical inert solvents that can be utilized in the process of this invention include halogenated hydrocarbons such as methylene dichloride, chloroform, carbon tetrachloride and chlorobenzene and aromatic hydrocarbons such as benzene, toluene and xylene.

A preferred process of this invention comprises using a hydrazone of Formula (III) where $R^1$ and $R^2$ are hydrogen, methyl and ethyl, and X is fluoro, chloro, bromo, iodo, trifluoromethyl, nitro, methyl, methoxy, methylthio, methylsulfonyl or methylsulfinyl. In this preferred process, there is used an alcohol of Formula (II) wherein R is methyl, ethyl, propyl, butyl, cyclohexyl or benzyl and there is used a desiccant which is trimethylorthoformate, triethylorthoformate, triisopropylorthoformate, tri-n-butlyorthoformate, trimethyl borate, triethyl borate, tetramethyl silicate, tetraethyl silicate, acetaldehyde dimethyl acetal, acetone dimethyl ketal, a Linde Type 4-A molecular sieve, or phosphorus pentoxide. In this preferred process the desiccant is selected in such a manner that any alcohol produced in the dehydrating process is the same as the alcohol utilized as the reagent ROH in the process. Finally, in this preferred process, there is used a catalytic amount of a strong acid which is sulfuric, hydrochloric or hydrobromic acid, and there is used an excess amount of the alcohol utilized as the reagent ROH as the reaction solvent and a temperature 40°C to 120°C.

Another feature of this invention comprises the additional step of cooling the resultant reaction

mixture, adding it to water and extracting the desired product with an appropriate solvent. Appropriate solvents which can be utilized in this extraction procedure include methylene chloride, chloroform, carbon tetrachloride, benzene, toluene, xylene and diethylether.

The isolated ester can, if desired, be treated with a dilute aqueous solution of an alkali metal hydroxide such as sodium hydroxide or potassium hydroxide to form the sodium or potassium salt of the respective oxopyridazine-3-carboxylic acid.

A particularly preferred process of this invention comprises reacting a hydrazone of Formula (I) as defined above, with an alcohol of Formula (II) as defined above in the prescence of a tri($C_1$-$C_4$)alkylorthoformate having the same alkyl substituent as the alkyl group of the alcohol ROH used in the reaction. In this process, it is also particularly preferred to use a catalytic amount of sulfuric acid and a temperature from $60^{\circ}C$ to $110^{\circ}C$.

The desiccants utilized in this invention are either commercially available or can be readily prepared. For example, trimethylorthoformate and triethylorthoformate are commercially available products. Other trialkylorthoformates can be readily prepared _in situ_ from these products by transesterification with the desired alcohol prior to the addition of the hydrazone.

The following examples are presented to illustrate the process of the present invention. In the examples, percentages are on a weight basis unless otherwise indicated and degrees are degrees Celsius.

Example 1 - Preparation of 1-phenyl-3-carbomethoxy-1,4-dihydro-4-oxo-6-methylpyridazine

8.7 g (0.0379 mole) 3-phenylhydrazono-4-oxo-6-methyl-2-pyrone, 100 ml anhydrous methanol, 10 g 96% sulfuric acid and 10 g (0.094 mole) trimethylorthoformate were refluxed for 5 minutes. The reaction mixture was cooled and poured into water. The aqueous mixture was extracted with methylene chloride. The organic layers were combined and backwashed with water. Removal of the solvent yielded 5.9 g of crude ester which was recrystallized from methylene chloride/ether (4.4 g, 47%, mp 200-2$^{o}$).

Example 2 - Preparation of 1-phenyl-3-carboethoxy-1,4-dihydro-4-oxo-6-methylpyridazine

8.7 g (0.0379 mole) 3-phenylhydrazono-4-oxo-6-methyl-2-pyrone, 100 ml anhydrous ethanol, 10 g 96% sulfuric acid and 14 g triethylorthoformate (0.0945 mole) were refluxed for 10 minutes. The reaction mixture was cooled, poured into water and extracted with methylene chloride. The organic layers were combined and backwashed with water. Removal of the solvent yielded 6.6 g (67%) of the desired ester. Mp (after recrystallization from acetone/ether) = 178-80$^{o}$

Example 3 - Preparation of 1-(4-chlorophenyl)-3-carbomethoxy-1,4-dihydro-4-oxo-6-methylpyridazine

A 1000 ml flask was charged with 400 ml anhydrous methanol, 40 g 96% sulfuric acid and 40 g (0.377 mole) of trimethylorthoformate. 20 g (0.076 mole) of 3-(4-chlorophenylhydrazono)-4-oxo-6-methyl-2-pyrone was added and the suspension was brought rapidly to reflux. The hydrazone dissolved fairly rapidly. After a total reflux time of 30 minutes the homogeneous reaction mixture was cooled and poured into cold water. The aqueous mixture was then extracted

with three portions of methylene chloride (3 x 200 ml). The organic extracts were combined and backwashed once with water. Removal of the organic solvent left 15.1 g of the desired ester (72%) mp = 200-2$^{\circ}$ after recrystallization from methylene chloride/ether.

Example 4 - Preparation of 1-(4-chlorophenyl)-3-carboethoxy-1,4-dihydro-4-oxo-6-methylpyridazine

10 g (0.0379 mole) of 3-(4-chlorophenylhydrazono)-4-oxo-6-methyl-2-pyrone, 100 ml anhydrous ethanol, 10 g 96% sulfuric acid and 14 g (0.0945 mole) of triethylorthoformate were combined in a suitable flask. The mixture was rapidly brought to reflux and maintained at that temperature for a total of 15 minutes. The homogeneous reaction mixture was then cooled and poured into 400 ml cool water. The resulting aqueous suspension was extracted with methylene chloride (2 x 100 ml). The organic extracts were combined and backwashed once with water. Removal of the solvent yielded 10.7 g of the desired ester (96%). Mp 143$^{\circ}$ (from methylene chloride/ether).

Example 5 - Preparation of 1-(4-chlorophenyl)-3-carbopropoxy-1,4-dihydro-4-oxo-6-methylpyridazine

Part One:

200 ml anhydrous *n*-propanol, 20 g 96% sulfuric acid and 20 g trimethylorthoformate (0.076 mole) were combined and charged into a flask fitted with a vigreaux column and a distillation head. The mixture was slowly warmed and all volatile substances boiling at or below 65$^{\circ}$C were collected and discarded (17 g total). The resulting reaction mixture, contains tri-*n*-propylorthoformate was cooled prior to the next step.

Part Two:

10 g (0.0379 mole) of 3-(4-chlorophenyl-hydrazono)-4-oxo-6-methyl-2-pyrone was charged to the above mixture. The resulting suspension was rapidly brought to reflux temperature (97°) and maintained for 5 minutes. The reaction mixture was then cooled as rapidly as possible and poured into 600 ml cold water. The aqueous mixture was extracted with 2 x 100 ml methylene chloride. The organic extracts were combined and backwashed once with water. Removal of the solvent yielded 13.5 g of a light brown oil that was crystallized from ether. Yield after crystallization is 6.9 g (mp 91-5°, 59%).

Example 6 - Preparation of 1-(4-chlorophenyl)-3-carbisopropoxy-1,4-dihydro-4-oxo-6-methylpyridazine

Part One:

250 ml anhydrous isopropanol, 20 g 96% sulfuric acid and 20 g (0.076 mole) trimethylorthoformate were charged to a flask fitted with a vigreaux column and a distillation head. The mixture was gently brought to reflux. All volatile substances boiling between 35° and 80° were collected and discarded (42 g total). The reaction mixture was then cooled to room temperature.

Part Two:

10 g of 3-(4-chlorophenylhydrazono)-4-oxo-6-methyl-2-pyrone (0.0379 mole) were suspended in the above mixture. The resulting suspension was rapidly brought to reflux (82°) and maintained at that temperature for 10 minutes. The mixture was cooled, poured into 300 ml water and extracted with 2 x 100 ml methylene chloride. The organic layers were combined and backwashed once with water. Removal of the solvent yielded 11.6 g of crude product which was

recrystallized from methylene chloride/ether to yield 9.4 g of desired ester (mp 147-9°, 81% yield).

Example 7 - Preparation of 1-(4-chlorophenyl)-3-carbobutoxy-1,4-dihydro-4-oxo-6-methylpyridazine

Part One:

200 ml anhydrous *n*-butanol, 20 g 96% sulfuric acid and 20 g (0.076 mole) trimethylorthoformate were charged to a flask fitted with a vigreaux column and a distillation head. The mixture was gently refluxed. All volatile substances boiling between 35-80°C were collected and discarded (23.2 g total). The residue containing tri-*n*-butylorthoformate was cooled to room temperature.

Part Two:

10 g of 3-(4-chlorophenylhydrazono)-4-oxo-6-methyl-2-pyrone (0.0379 mole) was added to the above mixture and the contents of the flask were heated to 80°C. As soon as the hydrazone dissolved (approx. 10 minutes) the mixture was cooled and poured into 300 ml water. The aqueous mixture was extracted with methylene chloride (2 x 150 ml). The organic layers were combined and backwashed with water several times. The organic layer was then backwashed with dilute $NaHCO_3$ solution followed by one more wash with water. The solvent was removed yielding 11.4 g of a brownish solid which was recrystallized from ether (7.4 g 61%, mp 89°).

As stated above the improved process of the present invention produces higher yields of purer product at much shorter reaction times than processes utilizing Fischer esterifications. Table I gives a comparison of the process of the present invention as compared to standard Fischer esterification techniques.

In this comparison, the data for the Fisher esterification were obtained utilizing the following

procedure making substitutions of appropriate starting materials and conditions listed in Table I below.

A suspension of 30 g of 1-phenyl-6-methyl-1, 4-dihydro-4-oxopyridazine-3-carboxylic acid (0.13 mole) was prepared in 240 ml of dry methanol. 36 g of 96% sulfuric acid was added. The suspension was refluxed for 20 hours. The resulting homogeneous dark brown solution was poured into 400 ml water and extracted with methylene chloride (3 x 100 ml). Evaporation of the solvent yielded a dark oil which crystallized when dissolved in methylene chloride/ether. Recrystallization of this solid from methylene chloride/ether yielded 17.2 g of the desired ester (mp 200-2.5$^{O}$) yield = 54%.

TABLE I

Present Process Versus Fischer Esterification Process

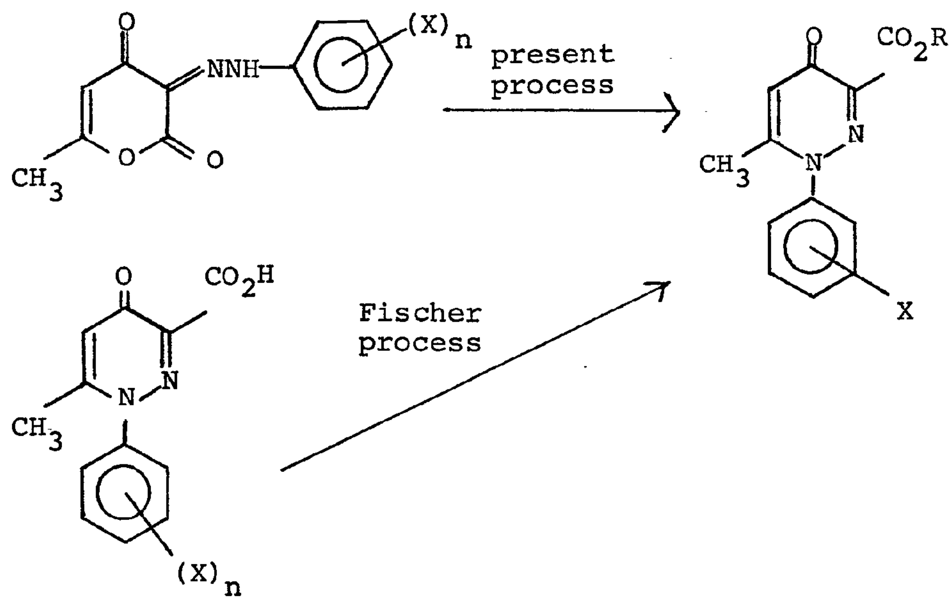


| R | X | Fischer Process Yield | Fischer Process Time (Temp.) | Present Process Yield | Present Process Time (Temp.) |
|---|---|---|---|---|---|
| $CH_3$ | H | 54 | 20 hrs. (reflux) | 47 | 5 minutes (reflux) |
| $C_2H_5$ | H | 35 | 20 hrs. (reflux) | 67 | 10 minutes (reflux) |
| $CH_3$ | 4-Cl | 45 | 10 hrs. (reflux) | 72 | 30 minutes (reflux) |
| $C_2H_5$ | 4-Cl | 60 | 72 hrs. ($20^o$) | 96 | 15 minutes (reflux) |

Claims:

1. A process for the preparation of a 1-aryl-1, 4-dihydro-4-oxopyridazine carboxylic acid ester of the formula:

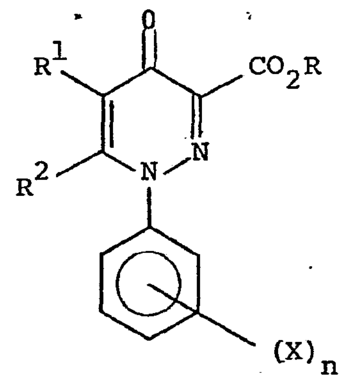

(I)

which comprises reacting, in a liquid medium and in the substantial absence of water available to undergo reaction to form undesired by-product(s), an alcohol with a hydrazone in the presence of (a) a strong acid catalyst and (b) a desiccant which serves to remove water of reaction, wherein the alcohol is of the formula ROH and the hydrazone is of the formula:

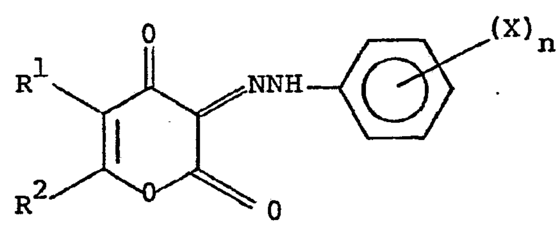

where in the above formulae:

R is $(C_1-C_6)$alkyl, $(C_1-C_4)$alkoxy$(C_1-C_4)$alkyl, $(C_3-C_8)$cycloalkyl, $(C_3-C_8)$cycloalkyl-$(C_1-C_4)$alkyl, halo$(C_1-C_6)$alkyl, benzyl or

benzyl the ring of which is substituted with up to two of the same or different substituents selected from chloro, bromo, methyl, ethyl, methoxy, cyano, nitro and trifluoromethyl;

$R^1$ and $R^2$ are selected from hydrogen and $(C_1-C_4)$alkyl;

X is selected from halo, trihalomethyl, nitro, $(C_1-C_4)$alkyl, $(C_1-C_4)$alkoxy, $(C_1-C_4)$alkylthio, $(C_1-C_4)$alkylsulfinyl, $(C_1-C_4)$alkylsulfonyl, cyano and carboxy, the X substituents being the same or different when n is $>1$; and n is 0 or an integer of from 1-3.

2. A process as claimed in Claim 1, wherein there is used at least one mole of the alcohol ROH per mole of hydrazone and the reaction is carried out in solution at a temperature from 25°C to 150°C.

3. A process as claimed in Claim 2, wherein the reaction is carried out at a temperature from 40°C to 120°C.

4. A process as claimed in any one of Claims 1-3, wherein there is used as acid catalyst sulfuric, hydrochloric, hydrobromic, nitric, perchloric, phosphoric, trifluoromethylacetic or methanesulfonic acid.

5. A process as claimed in any one of Claims 1-4, wherein the desiccant used is a tri$(C_1-C_4)$alkylorthoformate.

6. A process as claimed in Claim 5, wherein an excess of the alcohol ROH is used as reaction solvent.

7. A process as claimed in Claim 3 or 4, wherein the alcohol ROH employed is the same as that liberated by

the tri$(C_1-C_4)$alkylorthoformate in acting as desiccant.

8. A process as claimed in Claim 7, wherein $R^1$ and $R^2$ are hydrogen or methyl, X is chloro, bromo or iodo, R is $(C_1-C_4)$alkyl and the reaction is carried out at a temperature of from 60°C to 110°C.

9. A process as claimed in Claim 8 including the additional step of cooling the reaction mixture resulting from the process of Claim 8, adding it to water and extracting the desired product of Formula I with a solvent.

10. A process as claimed in any one of Claims 1-4, wherein there is used as desiccant a tri$(C_1-C_4)$alkyl borate, a tetra$(C_1-C_4)$alkyl silicate, a $(C_1-C_4)$alkyl acetal, a $(C_1-C_4)$alkyl ketal, a molecular sieve or phosphorus pentoxide.

11. A process as claimed in any preceding claim as applied to the preparation of 1-(4-chlorophenyl)-3-carbomethoxy-1,4-dihydro-4-oxo-6-methylpyridazine, 1-(4-chlorophenyl)-3-carboethoxy-1,4-dihydro-4-oxopyridazine, 1-(4-chlorophenyl)-3-carboisopropoxy-1,4-dihydro-4-oxopyridazine,1-(4-chlorophenyl)-3-carbo-*n*-butoxy-1,4-dihydro-4-oxopyridazine,1-phenyl-3-carbomethoxy-1,4-dihydro-4-oxopyridazine or 1-phenyl-3-carboethoxy-1,4-dihydro-4-oxopyridazine.